(19) Europäisches Patentamt
European Patent Office
Office européen des brevets

(11) Veröffentlichungsnummer: **0 022 504**
**B1**

(12) # EUROPÄISCHE PATENTSCHRIFT


(45) Veröffentlichungstag der Patentschrift: **16.03.83**

(51) Int. Cl.³: **C 07 H 15/22**, A 61 K 31/70

(21) Anmeldenummer: **80103698.9**

(22) Anmeldetag: **30.06.80**


(54) **1-N-Alkylisomicin-Derivate, Verfahren zu ihrer Herstellung und ihre Verwendung als Arzneimittel.**


(30) Priorität: **12.07.79 DE 2928183**

(43) Veröffentlichungstag der Anmeldung: **21.01.81 Patentblatt 81/3**

(45) Bekanntmachung des Hinweises auf die Patenterteilung: **16.03.83 Patentblatt 83/11**

(84) Benannte Vertragsstaaten: **AT BE CH DE FR GB IT LI NL**

(56) Entgegenhaltungen:
FR-A-2 240 015
FR-A-2 355 029


**Die Akte enthält technische Angaben, die nach dem Eingang der Anmeldung eingereicht wurden und die nicht in dieser Patentschrift enthalten sind.**


(73) Patentinhaber: **BAYER AG, Zentralbereich Patente, Marken und Lizenzen, D-5090 Leverkusen 1, Bayerwerk (DE)**

(72) Erfinder: **Voss, Eckart, Dr., Johann-Janssen-Strasse 38, D-5090 Leverkusen 1 (DE)**
Erfinder: **Metzger, Karl Georg, Dr., Pahlkestrasse 15, D-5600 Wuppertal 1 (DE)**
Erfinder: **Zeiler, Hans-Joachim, Dr., Windrather Strasse 188, D-5620 Velbert 15 (DE)**
Erfinder: **Petersen, Uwe, Dr., Auf dem Forst 4, D-5090 Leverkusen 1 (DE)**
Erfinder: **Stadler, Peter, Dr., Ohligserstrasse 85, D-5657 Haan (DE)**



EP 0 022 504 B1



Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

# 1-N-Alkylsisomicin-Derivate, Verfahren zu ihrer Herstellung und ihre Verwendung als Arzneimittel

Die vorliegende Erfindung betrifft neue Sisomicin-Derivate, Verfahren zu ihrer Herstellung sowie ihre Verwendung als Arzneimittel.

Sisomicin und seine bisher bekanntgewordenen Derivate gehören zur Klasse der Aminoglykosidantibiotika. Aminoglykosidantibiotika sind wichtige Substanzen zur wirkungsvollen Bekämpfung bakterieller Infektionen. Das Auftreten resistenter Keime mindert jedoch in vielen Fällen ihre breite Anwendbarkeit; des weiteren können Nebenwirkungen wie Oto- und Nephrotoxizitäten auftreten. Durch Derivatisierung gelingt es in einigen Fällen, diese Nachteile zu beseitigen. So sind unter anderem aus der DE-OS 2 437 160 bereits Aminoglykosidantibiotika bekanntgeworden, die an der 1-Aminogruppe durch $-CH_2X$-Reste substituiert sind.

Es wurde nun gefunden, dass die genannten Nachteile in besonders hohem Masse durch die erfindungsgemässen Verbindungen der allgemeinen Formel vermieden werden,

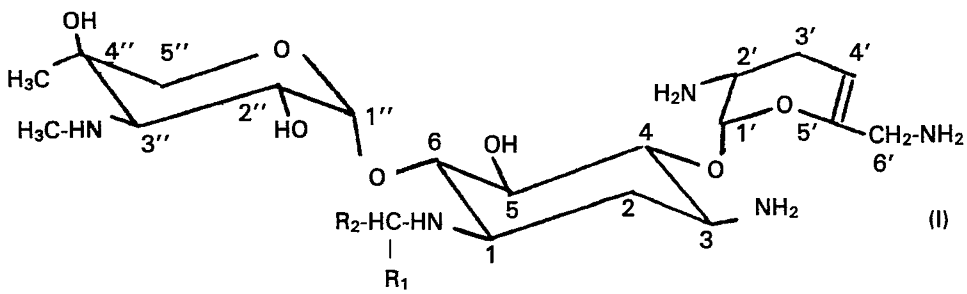

(I)

worin

$R^1$ einen durch Hydroxy, $C_1$-$C_4$-Alkoxy, Amino, $C_1$-$C_4$-Alkylamino, Di-$C_1$-$C_4$-Alkylamino, Acyloxy, Acylamino, N-$C_1$-$C_4$-Alkyl-N-acylamino, Carboxy, $C_1$-$C_4$-Alkoxycarbonyl, Aminocarbonyl, $C_1$-$C_4$-Alkylaminocarbonyl oder Di-$C_1$-$C_4$-Alkylaminocarbonyl substituierten $C_1$-$C_6$-Alkylrest und

$R^2$ einen gegebenenfalls durch Hydroxy, $C_1$-$C_4$-Alkoxy, Amino, $C_1$-$C_4$-Alkylamino, Di-$C_1$-$C_4$-Alkylamino, Acyloxy, Acylamino, N-$C_1$-$C_4$-Alkyl-N-acylamino, Carboxy, $C_1$-$C_4$-Alkoxycarbonyl, Aminocarbonyl, $C_1$-$C_4$-Alkylaminocarbonyl oder Di-$C_1$-$C_4$-Alkylaminocarbonyl substituierten $C_1$-$C_6$-Alkylrest bedeuten, oder

$R^2$-HC-$R^1$ (mit freier Bindung am C) für einen Piperidin-4-yl- oder 4-Hydroxycyclohex-1-yl-Rest steht,

mit der Bedingung, dass der Rest $R^1$-CH-$R^2$ (mit freier Bindung am C) nicht gleichzeitig Amino- und Hydroxygruppen als Substituenten tragen kann.

In der Substituentendefinition der Alkylreste $R^1$ und $R^2$ steht Acyl insbesondere für $C_1$-$C_4$-Alkylcarbonyl.

Vorzugsweise bedeutet $R_1$ durch Hydroxy substituiertes $C_1$-$C_6$-Alkyl, insbesondere Methyl und Hydroxymethyl und $R^2$ gegebenenfalls ein- bis fünfmal durch Hydroxy oder Carboxy substituiertes $C_1$-$C_6$-Alkyl.

In FR-A 2 240 015 werden zahlreiche 1-N-substituierte Sisomicin-Derivate beschrieben, wobei jedoch nichts über sekundäre Alkylgruppen und insbesondere nichts über substituierte sekundäre Alkylgruppen als mögliche Substituenten ausgesagt wird. Von den in dieser Literaturstelle beschriebenen Verbindungen hat nur jene von Beispiel 1A (1-N-Ethyl-Sisomicin) zum Handelsprodukt geführt, woraus zu schliessen ist, dass diese Verbindung von den zahlreichen in FR-A 2 240 015 genannten Aminoglycosid-Derivaten als die beste anzusehen ist. Wie sich zeigte, weisen die erfindungsgemässen Verbindungen gegenüber diesem Produkt jedoch eine deutlich verbesserte antibiotische Wirksamkeit auf.

FR-A 2 355 029 betrifft im wesentlichen Kanamycin-Derivate. Als mögliche Substituenten am 1-N-Atom werden zwar neben primären auch sekundäre durch Hydroxy substituierte Alkylreste erwähnt; es ist jedoch nicht ersichtlich, dass sekundäre Alkylreste eine Verbesserung der antibiotischen Wirkung gegenüber primären Alkylgruppen bewirken. Es muss daher als überraschend angesehen werden, dass dies im Falle des Sisomicins eintritt. Darüber hinaus bringt die Substitution an mindestens einem der Reste $R_1$ und $R_2$, wie Vergleichsversuche zeigten, eine weitere Verbesserung der antibiotischen Wirksamkeit.

Die erfindungsgemässen Verbindungen sowie ihre pharmazeutisch verwendbaren Salze zeigen starke antibakterielle Eigenschaften gegen eine Vielzahl von Keimen und eine aussergewöhlich gute Verträglichkeit.

Die pharmazeutisch verwendbaren Salze leiten sich insbesondere von anorganischen oder organischen Säuren wie Schwefelsäure, Phosphorsäure, Salpetersäure, Salzsäure, Bromwasserstoffsäure, Essigsäure, Propionsäure, Ascorbinsäure, Zitronensäure usw. ab.

Geeignete Reste $R_1$ und $R_2$ sind beispielsweise 1-Hydroxyprop-2-yl, 4-Hydroxybut-2-yl, 5-Hydroxypent-2-yl, 4-Hydroxycyclohex-1-yl, 3,4-Dihydroxybut-2-yl, 4,5-Dihydroxypent-2-yl, 1,3-Dihydroxyprop-2-yl, 1,3,4-Trihydroxybut-2-yl, 1,3,4,5-Tetrahydroxypent-2-yl, 1,3,4,5,6-Pentahydroxyhex-2-yl, Piperidin-4-yl, 1-aminoprop-2-yl, 4-Aminobut-2-yl, 5-Aminopent-2-yl, 1,7-Diaminohept-4-yl. Ein geeigneter Rest $R_2$ ist auch Prop-2-yl.

Die vorstehend aufgeführten Reste sind lediglich beispielhaft zu verstehen. Sie enthalten meistens mindestens ein oder mehrere chirale C-Atome. Es

kann von Vorteil sein, die erfindungsgemässen Verbindungen als optisch reine Produkte herzustellen und zu verwenden.

Die erfindungsgemässen Verbindungen werden erhalten, indem man selektiv acylierte oder sulfenylierte Verbindungen der Formel

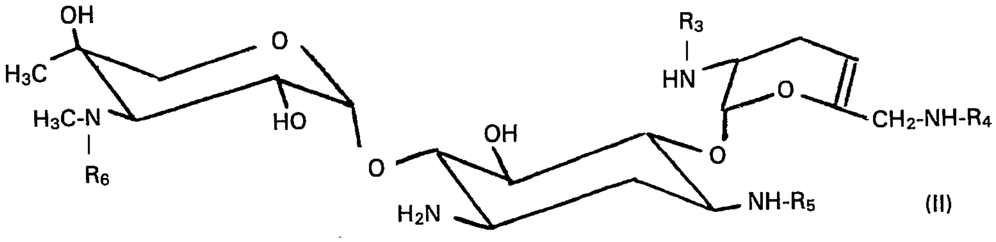

(II)

worin

$R_3$, $R_4$, $R_5$ und $R_6$ -SR' oder CO-R'' bezeichnen, wobei

R' gegebenenfalls substituiertes Phenyl, Di- oder Triphenylmethyl und

R'' $-(CH_2)_{n_1}$-B oder 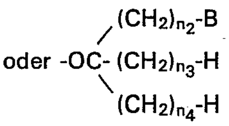

worin

B Wasserstoff oder gegebenenfalls substituiertes Phenyl bedeuten und

$n_1$, $n_2$, $n_3$ und $n_4$ unabhängig voneinander für eine Zahl von 0 bis 5 stehen,

mit einer Carbonylverbindung der Formel

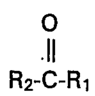

(III)

worin

$R_1$ und $R_2$ die oben angegebene Bedeutung haben und wobei Amino und Alkylamino als Substituenten von $R_1$ und $R_2$ durch R'-S- oder R''-CO-Gruppen geschützt sind, in Gegenwart eines Wasserstoff-Donor-Reduktionsmittels umsetzt und anschliessend die Schutzgruppen -S-R' oder -CO-R'' abspaltet.

Gegebenenfalls substituiertes Phenyl R' ist insbesondere Phenyl oder durch 1 bis 3 Substituenten aus der Reihe Nitro, $C_1$-$C_4$-Alkyl, $C_1$-$C_4$-Alkoxy, $C_1$-$C_4$-Alkoxycarbonyl oder Phenyl oder 1 bis 5 Halogenatome substituiertes Phenyl.

Gegebenenfalls substituiertes Phenyl B ist insbesondere Phenyl oder durch 1 oder 2 Substituenten aus der Reihe Nitro, $C_1$-$C_4$-Alkoxy, Phenyl oder Halogen substituiertes Phenyl.

Als Ausgangsstoffe der Formel (II) dienen bevorzugt 2',3,3'',6'-Tetra-N-(o-nitrophenylsulfenyl)-sisomicin, 3''-N-(o-Nitrophenylsulfenyl)-2',3,6'-tris-N-trichloracetylsisomicin, 3''-N-(o-Nitrophenylsulfenyl)-2',3,6'-tris-N-trifluoracetylsisomicin, 3''-N-(o-Nitrophenylsulfenyl)-2',3,6'-tris-N-(2,2,2-trichlorethoxycarbonyl)-sisomicin, 3''-N-(o-Nitrophenylsulfenyl)-2',3,6'-tris-N-(1,1-dimethyl-2,2,2-trichlorethoxycarbonyl)-sisomicin, 3''-N-(o-Nitrophenylsulfenyl)-2',3,6'-tris-N-(4-methoxybenzyloxycarbonyl)-sisomicin, 3''-N-(o-Nitrophenylsulfenyl)-2',3,6'-tris-N-phenoxycarbonylsisomicin, 3''-N-(o-Nitrophenylsulfenyl)-2',3,6'-tris-N-(tert.-butoxycarbonyl)-sisomicin, deren Herstellung nach dem in DE-OS 2 726 197 beschriebenen Verfahren, bzw. über die folgenden Stufen erfolgt:

1) Umsetzung von Penta-N-(o-nitrophenylsulfenyl)-sisomicin (DE-OS 2 726 197) mit Dimethyl-(1,2-dimethylpropyl)silylchlorid zum Penta-N-(o-nitrophenylsulfenyl)-2''-O-[dimethyl-(1,2-dimethylpropyl)-silyl]-sisomicin;

2) Abspaltung der o-Nitrophenylsulfenylgruppen von 2',3,6'-N mit 2-Mercaptobenzthiazol;

3) Acylierung der 2',3,6'-Positionen mit einem üblichen Acylierungsmittel;

4) Abspaltung der 2''-O-Schutzgruppe und

5) Abspaltung der 1-N-(o-Nitrophenylsulfenyl)-Gruppe.

Die reduktive Alkylierung mit einer Carbonylverbindung der Formel (III) in Gegenwart eines Wasserstoff-Donor-Reduktionsmittels wird gewöhnlich bei Raumtemperatur in Gegenwart von Luft durchgeführt, obwohl es günstiger sein kann, die Reaktion unter Inertgas (Argon, Stickstoff) durchzuführen. Die Reaktion ist gewöhnlich sehr rasch, oft in weniger als 60 Minuten vollendet, was durch dünnschichtchromatographische Bestimmungen festgestellt werden kann.

Wasserstoff-Donor-Reduktionsmittel, die bei diesem Verfahren Verwendung finden, umfassen Dialkylaminborane, z.B. Dimethylaminboran, Diethylaminboran, und Morpholinboran, Tetraalkylammoniumcyanohydride, z.B. Tetrabutylammoniumcyanoborhydrid, Alkalimetallborhydride, z.B. Natriumborhydrid und vorzugsweise Alkalimetallcyanoborhydride wie Lithiumcyanoborhydrid und Natriumcyanoborhydrid.

Das Verfahren wird üblicherweise in einem inerten Lösungsmittel durchgeführt. Das Lösungsmittel kann ein organisches oder anorganisches sein, in dem das selektiv geschützte Sisomicin und die anderen Reagenzien löslich sind und das unter den Reaktionsbedindungen nach Möglichkeit Nebenreaktionen herabsetzt oder verhindert. Obwohl wasserfreie aprotische Lösungsmittel mit Vorteil eingesetzt werden können, z.B. Tetrahydrofuran, wenn das Reduktionsmittel Morpholinboran ist, wird gewöhnlich doch ein protisches Lösungsmittel verwendet. Als soches eignet sich z.B. ein niederes Alkanol oder vorzugsweise Wasser oder ein wässriges niederes Alkanol, vorzugsweise wässriges Methanol, Ethanol oder Aceton oder andere Lösungsmittelsysteme, die Wasser enthalten wie wässriges Dimethylformamid, wässriges Hexamethylphosphoramid, wässriges Tetrahydrofuran oder wässriger Ethylenglykoldimethylether.

Das Verfahren wird gewöhnlich in einem pH-Bereich von 1 bis 11 und vorzugsweise 4 bis 8 durchgeführt.

Die bei dem Verfahren verwendeten Carbonylverbindungen sind entweder bekannt oder durch bekannte Synthesen zugänglich. Carbonylverbindungen mit chiralen C-Atomen stehen teilweise, insbesondere, wenn es sich um Ketozucker handelt, als optisch einheitliche Verbindungen zur Verfügung. Sie können entweder in freier Form oder auch als Ketale, z.B. als Dimethylketale, zur reduktiven Alkylierung eingesetzt werden. Man arbeitet bei der Verwendung von Ketalen in Gegenwart von Mineralsäuren oder organischen Säuren wie Essigsäure, wodurch das Ketal gespalten wird und die freigesetzte Carbonylverbindung sofort mit der entsprechenden Aminogruppe des Sisomicins reagiert.

Als Beispiele für die erfindungsgemässen Wirkstoffe seien im einzelnen genannt: 1-N-(1-Hydroxyprop-2-yl)-sisomicin, 1-N-(4-Hydroxybut-2-yl)-sisomicin, 1-N-(5-Hydroxypent-2-yl)-sisomicin, 1-N-(4--Hydroxycyclohex-1-yl)-sisomicin, 1-N-(3,4-Dihydroxybut-2-yl)-sisomicin, 1-N-(4,5-Dihydroxypent-2-yl)-sisomicin, 1-N-(1,3-Dihydroxyprop-2--yl)-sisomicin, 1-N-(1,3,4-Trihydroxybut-2-yl)-sisomicin, 1-N-(1,3,4,5-Tetrahydroxypent-2-yl)-sisomicin, 1-N-(1,3,4,5,6-Pentahydroxyhex-2-yl)-sisomicin, 1-N-(Piperidin-4-yl)-sisomicin, 1-N-(A-aminoprop-2-yl)-sisomicin, 1-N-(4-Aminobut-2-yl)-sisomicin, 1-N-(5-Aminopent-2-yl)-sisomicin und 1-N--(1,7-Diaminohept-4-yl)-sisomicin.

Die erfindungsgemässen Verbindungen sind antimikrobielle Mittel mit einem breiten Wirkungsspektrum und besonderer Wirksamkeit gegen gram-negative Bakterien. Diese Eigenschaften ermöglichen ihre Verwendung als Arzneimittel, insbesondere bei der Bekämpfung von durch Bakterien hervorgerufenen Erkrankungen bei Mensch und Tier.

Sie sind gut zur Prophylaxe und Chemotheraphie von lokalen und systemischen Infektionen, insbesondere Infektionen des Urogenitalsystems in der Human- und Tiermedizin, geeignet, die durch gramnegative Bakterien, z.B. E.coli, Proteus, Klebsiella und Pseudomonas verursacht werden. Hemmhöfe im Agar-Lochtest wurden z.B. gegen folgende Bakterienstämme bei einer Konzentration von 100 Mikrogramm/1 ml gefunden:

| | |
|---|---|
| Pseudomonas aerug. | 5737 |
| Pseudomonas aerug. | F 41 |
| Klebsiella pneum. | 2 München |
| Klebsiella pneum. | 1 Düsseldorf |
| E. coli | Münster |
| E. coli | Neumann |

Das Verfahren zur Behandlung von durch Bakterien hervorgerufenen Erkrankungen ist dadurch gekennzeichnet, dass man die erfindungsgemässen Verbindungen Menschen und Tieren appliziert, die an diesen Erkrankungen leiden.

Im allgemeinen hängt die zu verabreichende Dosis der Verbindungen der Erfindung vom Alter und Gewicht des Lebewesens, von der Verabreichungsart, vom Typ und von der Schwere der bakteriellen Infektion ab. Die Dosierung der erfindungsgemässen Verbindungen ist gewöhnlich der Dosierung der 1-N-unsubstituierten Verbindungen ähnlich. Der Dosierungsspielraum beträgt von 20 mg/Tag/Mensch bis 2000/mg/Tag/Mensch, vorzugsweise 100 mg bis 500 mg/Tag.

Die Verbindungen der Erfindung können oral verabreicht werden. Die Verabreichung kann in Einzelgaben oder auf mehreren Gaben verteilt erfolgen. Sie können auch topisch verabreicht werden in Form von Salben, Creme oder Lotionen. Pharmazeutische Trägerstoffe für diese Formulierungen umfassen Wasser, Öle, Fette, Polyester und Polyole.

Wie im folgenden gezeigt wird ist das Verfahren zur Herstellung von Arzneimitteln, welche die erfindungsgemässen Produkte enthalten, dadurch gekennzeichnet, dass man die neuen Verbindungen mit pharmazeutisch geeigneten Träger- und/oder Zusatzstoffen vermischt.

Zur oralen Verabreichung der Verbindungen dieser Erfindung können Tabletten, Kapseln oder Elixiere Anwendung finden, die Verbindungen können aber auch dem Tierfutter zugemischt werden.

Im allgemeinen enthalten topische Präparationen ca. 0,1 bis ca. 3,0 g der Verbindungen der Erfindung pro 100 g Salbe, Creme oder Lotion. Die topische Verabreichung erfolgt ca. 2- bis 5mal am Tag.

Die antibakterien Mittel der Erfindung können in flüssiger Form als Lösungen oder Suspensionen zur Anwendung an Ohren und Augen oder zur parenteralen Verabreichung in Form intramuskulärer Injektionen vorliegen. Injektionslösungen oder -suspensionen werden gewöhnlich so verabreicht, dass ca. 1 bis 15 mg Wirkstoff pro Kilogramm Körpergewicht in 2 bis 4 Dosen pro Tag in den infizierten Organismus gelangen. Die genaue Dosis hängt von der Art der Infektionen, der Empfindlichkeit des infizierten Keims und von den individuellen Charakteristika des zu Behandelnden ab.

Formulierung 1

| Tablette | 10 mg Tab. | 100 mg Tab. |
|---|---|---|
| a) 1-N-[1-Hydroxy-prop-2-yl]--sisomicin | 10,50*mg | 102,50*mg |
| Lactose | 197,50 mg | 126,00 mg |
| Maisstärke | 25,00 mg | 35,00 mg |
| Polyvinylpyrrolidon | 7,50 mg | 7,50 mg |
| Magnesiumstearat | 2,50 mg | 3,50 mg |

*5%iger Überschuss

Zur Herstellung der Tabletten wird eine Aufschlämmung des betreffenden Wirkstoffs, Lactose und Polyvinylpyrrolidon, hergestellt und diese sprühgetrocknet. Man gibt die Maisstärke und Magnesiumstearat zu und verpresst zu Tabletten.

Formulierung 2

Salbe

| | |
|---|---|
| 1-N-[1-Hydroxyprop-2-yl]-sisomicin | 1,0 g |
| Methylparaben U.S.P. | 0,5 g |
| Propylparaben U.S.P. | 0,1 g |
| Petrolatum | auf 1000 g |

Herstellung
(1) Man schmilzt das Petrolatum;
(2) mischt den Wirkstoff Methylparaben und Propylparaben mit ca. 10% des geschmolzenen Petrolatum,
(3) gibt das Gemisch in eine Kolloidmühle,
(4) gibt das restliche Petrolatum unter Rühren zu und kühlt, bis es halbfest wird. Das Produkt wird in geeignete Behälter abgefüllt.

Formulierung 3

| Injektionslösung | Per 2,0 ml Viole | per 50 Liter |
|---|---|---|
| 1-N-[1-Hydroxyprop--2-yl]-sisomicin | 84,0 mg* | 2100,0 gm |
| Methylparaben, U.S.P. | 3,6 mg | 90,0 gm |
| Propylparaben, U.S.P. | 0,4 mg | 10,0 gm |
| Natriumbisulfit, U.S.P. | 6,4 mg | 160,0 gm |
| Dinatriumäthylendiamintetraacetat-dihydrat | 0,2 mg | 5,0 mg |
| Wasser, U.S.P. q.s. | 2,0 mg | 50,0 Liter |

*5%iger Überschuss

*Beispiel 1*

*Penta-N-(o-nitrophenylsulfenyl)-2''-O-[dimethyl--(1,2-dimethyl-propyl)-silyl]-sisomicin*

60,6 g rohes Penta-N-(o-nitrophenylsulfenyl)-sisomicin und 8,75 g Imidazol werden in 250 ml absolutem Dichlormethan gelöst. Bei 0°C werden unter Feuchtigkeitsausschluss 22,5 ml Dimethyl-(1,2-dimethyl-propyl)-silylchlorid zugetropft. Der Ansatz wird im Vakuum auf ca. 170 ml eingedampft und bei Raumtemperatur 48 Stunden stehen gelassen. Nach Zugabe von 130 ml absolutem Dichlormethan wird der Niederschlag abgesaugt, das Filtrat mit 350 ml Petroläther kräftig durchgeschüttelt und die Petrolätherphase abdekantiert und verworfen. Das ausgefallene Öl wird in 100 ml Dichlormethan glöst, mit 250 ml Petroläther erneut ausgefällt und schliesslich im Hochvakuum getrocknet. Ausbeute 60 g (89%) Rohprodukt, das ohne weitere Reinigung für die weiteren Umsetzungen eingesetzt wird. Ein reines Präparat wird durch Chromatographie an Kieselgel mit $CH_2Cl_2/CH_3OH$ = 99/1 erhalten.
$R_f$ ($CH_2Cl_2/CH_3OH$ = 99,5/0,5) = 0,62
13 - C - NMR ($CDCl_3$)
$\delta$ = 124-138 (arom. C); 147,54 (C-5'); 102,26 (C-1''); 97,81 (C-4'); 99,09 (C-1'); -2,9 bis -3,0 ($Si-CH_3$); 22,77 (Si-CH-); 30,60 (Si-CH-CH) ppm.

Als Nebenprodukt wird das Penta-N-(o-nitrophenylsulfenyl)-2'',5-bis-O-[dimethyl-(1,2-dimethyl-propyl)-silyl]-sisomicin isoliert.
$R_f$ ($CH_2Cl_2/CH_3OH$ = 99,5/0,5) = 0,79
13 - C - NMR ($CDCl_3$)
$\delta$ = 124-146 (arom. C), 148,00 (C-5'); 96,13 (C-4') ppm.

*Beispiel 2*

*1,3''-Bis-N-(-o-nitrophenylsulfenyl)-2''-O-[dimethyl-(1,2-dimethyl-propyl)-silyl]-sisomicin*

56 g rohes Penta-N-(-o-nitrophenylsulfenyl)-2''--O-[dimethyl-(1,2-dimethyl-propyl)-silyl]-sisomicin in 36 ml Dichlormethan/70 ml Methanol werden mit 16 g 2-Mercapto-benzthiazol versetzt, geschüttelt, bis klare Lösung erfolgt und bei 5°C 2 Stunden stehen gelassen. Der sich dabei abscheidende Niederschlag wird abfiltriert und die Lösung ohne Isolierung des gewünschten Produktes für die weiteren Umsetzungen verwendet. Die Ausbeute beträgt etwa 80% der Theorie. Zur Darstellung eines reinen Präparats wird das Filtrat rasch im Vakuum eingedampft und der Rückstand mit a) Dichlormethan, b) Dichlormethan/$CH_3OH$ (8:2) und c) mit $CH_2Cl_2/CH_3OH$/20% wässrigem Ammoniak (7:2,7:0,3), an Kieselgel chromatographiert. Die Ausbeute an reinem Produkt beträgt 25,3 g (69%).
$R_f$ ($CH_2Cl_2/CH_3OH$/20% wässriges $NH_3$ = 7:2,7:0,3) = 0,66
13 - C - NMR ($CD_3OD$)
$\delta$ = 1,5 ($Si-CH_3$); 122-146 (arom. C); 147,14 (C-5'); 103,31 (C-1''); 100,16 (C-1'); 99,30 (C-4') ppm.

Als Nebenprodukt werden bei der Säulenchromatographie 3 g (10%) 3''-N-(-o-Nitrophenylsulfenyl)--2''-O-[dimethyl-(1,2-dimethyl-propyl)-silyl]-sisomicin isoliert.
$R_f$ ($CH_2Cl_2/CH_3OH$/20% wässriges $NH_3$ = 7:2,7:0,3) = 0,15
13 - C - NMR ($CD_3OD$)
$\delta$ = 76,66 (C-2''); 21,70 (C-6''); 30,40 ($N-CH_3$); 53,13 (C-1); 52,18 (C-3); 44,06 (C-6'); 49,41 (C-2') ppm.

*Beispiel 3*

*1,3''-Bis-N-(-o-nitrophenylsulfenyl)-2',3,6'-tris-N--tri-chloracetyl-2''-O-[dimethyl-(1,2-dimethyl-propyl)-silyl]-sisomicin*

8,8 g 1,3''-Bis-N-(-o-nitrophenylsulfenyl)-2''-O--[dimethyl-(1,2-dimethyl-propyl)-silyl]-sisomicin in 20 ml Dichlormethan/20 ml Pyridin werden bei −15°C tropfenweise mit 7,5 ml Trichloressigsäureanhydrid versetzt und noch 10 Minuten bei Raumtemperatur weitergerührt. Nach Zugabe von 20 ml Dichlormethan wird der Ansatz zweimal mit je 20 ml $H_2O$ ausgeschüttelt, die organische Phase eingedampft und der Rückstand als Rohprodukt weiterverarbeitet.
$R_f$ ($CH_2Cl_2(CH_3OH$ = 97,5/2,5) = 0,72

*Beispiel 4*

*1,3''-Bis-N-(-o-nitrophenylsulfenyl)-2',3,6'-tris-N--trichloracetyl-sisomicin*

Das rohe Öl von Beispiel 3 wird in 20 ml Dimethylsulfoxid gelöst, mit 2 ml einer 50 proz. KF-Lösung versetzt und 3 Stunden kräftig gerührt. Das Produkt wird mit Wasser ausgefällt, mit Wasser gewaschen und getrocknet. Das Rohprodukt wird ohne weitere Reinigung weiterverarbeitet.

$R_f$ ($CH_2Cl_2/CH_3OH$ = 97,5/2,5) = 0,42
13 - C - NMR ($CDCl_3$)
δ = 103,60 (C-1''); 66,48 (C-3''); 55,15 (C-1); 50,60 (C-3); 79,86 (C-4); 76,18 (C-5); 89,16 (C-6); 97,74 (C-1'); 96,84 (C-4'); 149,80 (C-5); 92,78 ($CCl_3$); 162,29 und 162,11 (CO) ppm.

*Beispiel 5*

*3''-N-(-o-nitrophenylsulfenyl)-2',3',6'-tris-N-trichloracetyl-sisomicin*

Das Produkt von Beispiel 4 wird in 13 ml Dichlormethan gelöst, mit 26 ml Methanol und 5 g 2-Mercaptobenzthiazol bis zur klaren Lösung geschüttelt und 3 Tage bei 5°C stehen gelassen. Der Niederschlag wird abfiltriert, das Filtrat eingedampft und an Kieselgel chromatographiert (Laufmittel a: $CH_2Cl_2/CH_3OH$ = 95/5; b: ($CH_2Cl_2/CH_3OH$/20% wässriges $NH_3$ = 93/6,5/0,5).
$R_f$ ($CH_2Cl_2/CH_3OH$/20% wässriges $NH_3$ = 93/6,5/0,5) = 0,43
13 - C - NMR ($CDCl_3$)
δ = 103,43 (C-1''); 67,46 (C-3''); 50,85 (C-1); 50,28 (C-3); 79,44 (C-4); 76,51 (C-5); 89,29 (C-6); 97,61 (C-1'); 96,62 (C-4'); 149,50 (C-5'); 92,46 und 92,38 (C-4'); 162,01 und 161,76 (CO) ppm.

*Beispiel 6*

*1-N-(1-Hydroxyprop-2-yl)-sisomicin*

10 g 3''-N-(-o-Nitrophenylsulfenyl)-2',3,6'-tris-N-trichloracetylsisomicin werden in 50 ml Tetrahydrofuran und 15 ml Wasser gelöst, mit 1,3 ml Hydroxyaceton und portionsweise mit 1,5 g $NaBH_3CN$ versetzt und mit Essigsäure auf pH 5 gestellt. Nach 20 Stunden bei Raumtemperatur wird der Ansatz im Vakuum eingedampft,der Rückstand in Dichlormethan aufgenommen und mit Wasser mehrfach ausgeschüttelt.

Die organische Phase wird auf 50 ml eingeengt, mit 50 ml gesättigter methanolischer Bariumhydroxid-Lösung sowie 30 ml Methanol versetzt und bei Raumtemperatur 10 Stunden stehen gelassen. Nach Abdampfen des Lösungsmittels im Vakuum wird der Rückstand mit 3,4 g 2-Mercapto-benzthiazol in 20 ml Dichlormethan/6 ml Methanol versetzt und mit 4n Schwefelsäure angesäuert. Die wässrige Phase wird abgetrennt, der Niederschlag abzentrifugiert, das Zentrifugat mit basischem Ionenaustauscher ($OH^-$-Form) auf pH 10 gebracht, eingedampft und an Kieselgel chromatographiert (Laufmittel: $CH_2Cl_2/CH_3OH$/20% wässriges $NH_3$ = 2/4/1). Die Hauptkomponente wird durch Eindampfen der entsprechenden Fraktionen isoliert.
$R_f$ = 0,4 ($CH_2Cl_2/CH_3OH$/20% wässriges $NH_3$ = 2/4/1).

*Beispiel 7*

*1-N-(1,3-Dihydroxyprop-2-yl)-sisomicin*

1,5 g 3''-N-(o-Nitrophenylsulfenyl)-2',3,6'-tris-N-trichloracetylsisomicin werden in 15 ml Methanol/2,4 ml Wasser gelöst und mit Essigsäure auf pH 5,5 gebracht. Nach Zugabe von 250 mg $NaBH_3CN$ und 300 mg Dihydroxyaceton wird 5 Stunden bei Raumtemperatur gerührt und der Ansatz im Vakuum eingedampft. Der Rückstand wird in 5,3 ml Methanol aufgenommen und unter starkem Rühren in 53 ml Wasser eingetropft. Der Niederschlag wird abzentrifugiert, mit 20 ml Wasser digeriert, erneut abzentrifugiert, bei 45°C in 25 ml Methanol gelöst und mit 1,35 g $Ba(OH)_2$ in 7,5 ml Wasser versetzt. Nach 15 Stunden bei Raumtemperatur wird der Ansatz mit 7,5 ml Wasser verdünnt und die Bariumsalze durch Zugabe von festem $CO_2$ gefällt. Der Niederschlag wird abgesaugt, das Filtrat eingedampft und der Rückstand an Kieselgel chromatographiert (Laufmittel: Unterphase des Gemisches von $CH_2Cl_2/CH_3OH$/15% $NH_3$ = 1/1/1).

Die orangefarbene Fraktion wird eingedampft, der Rückstand in 8 ml Dichlormethan/5 ml Methanol gelöst, mit 510 mg 2-Mercaptobenzthiazol in 1,5 ml $CH_3OH$/2,5 ml $CH_2Cl_2$ versetzt und mit 10% wässriger HCl auf pH 1 gebracht. Nach Zugabe von 14 ml $H_2O$ und 3,5 ml $CH_2Cl_2$ wird die Wasserphase abgetrennt, mit basischem Ionenaustauscher auf pH 7 gebracht, durch Zugabe von weiterem Ionenaustauscher auf pH 11 gebracht, der Ionenaustauscher abfiltriert und das Filtrat eingedampft. Das Produkt bleibt als farbloser Schaum zurück.
$R_f$ = 0,31 ($CH_2Cl_2/CH_3OH$/20% wässriges $NH_3$ = 2/4/1).

*Beispiel 8*

*1-N-(5-Hydroxypent-2-yl)-sisomicin*

1 g 3''-N-(o-Nitrophenylsulfenyl)-2',3,6'-tris-N-trichloracetylsisomicin wird in 5 ml Tetrahydrofuran und 1,5 ml Wasser gelöst und mit 220 mg 5-Hydroxypentanon-2 sowie 200 g $NaBH_3CN$ versetzt. Nach Einstellen von pH 5 mit Essigsäure wird der Ansatz 8 Stunden auf 50°C erhitzt, dann eingedampft, der Rückstand in Dichlormethan aufgenommen und mit Wasser zweimal ausgeschüttelt. Die organische Phase wird auf 5 ml eingeengt, mit 5 ml $CH_3OH$ sowie 5 ml gesättigter methanolischer $Ba(OH)_2$-Lösung versetzt und 12 Stunden bei Raumtemperatur stehen gelassen. Nach Neutralisation mit festem $CO_2$ wird der Niederschlag abfiltriert und das Filtrat an Kieselgel chromatographiert [Laufmittel: $CH_2Cl_2/CH_3$ = 8/2, unter Zugabe von steigenden Mengen (20 - 50%) des Gemisches $CH_2Cl_2/CH_3OH$/20% wässriges $NH_3$ = 2/4/1].

Die orangefarbene Hauptfraktion wird eingedampft, mit 2,1 g 2-Mercaptobenzthiazol in 7,5 ml $CH_3OH$/12,5 ml $CH_2Cl_2$ versetzt und mit 4n $H_2SO_4$ angesäuert. Nach Zugabe von 5 ml Wasser zum Reaktionsgemisch wird die wässrige Phase abgetrennt, zweimal mit je 1,5 ml $CH_2Cl_2$ extrahiert und mit basischem Ionenaustauscher auf pH 11 gebracht. Nach Eindampfen der wässrigen Lösung bleibt das Produkt als farbloser Schaum zurück.
$R_f$ = 0,48 ($CH_2Cl_2/CH_3OH$/20% wässriges $NH_3$ = 2/4/1).

*Beispiel 9*

*1-N-(4-Hydroxybut-2-yl)-sisomicin*

110 mg 2',3,3'',6'-Tetra-N-(o-nitrophenylsulfenyl)-sisomicin in 1,5 ml Tetrahydrofuran und 0,45 ml

Wasser werden mit 250 mg 4-Hydroxybutanon-2 sowie mit 12,5 mg $NaBH_3CN$ versetzt und mit Essigsäure auf pH 5 gebracht. Nach 30 Minuten wird der Ansatz eingedampft und die orangefarbene Hauptfraktion durch Chromatographie an Kieselgel (Laufmittel: $CH_2Cl_2/CH_3OH$ = 99/1) und Abdampfen des Eluierungsmittels isoliert. Zur Schutzgruppenabspaltung wird die Substanz in 0,6 ml $CH_2Cl_2$ gelöst und mit 68 mg 2-Mercaptobenzthiazol in 0,24 ml $CH_3OH$ und 0,4 ml $CH_2Cl_2$ versetzt, der Ansatz mit konz. $HCl/CH_3OH$ = 1/1 auf pH 1 gebracht, mit 3 ml Wasser durchgeschüttelt, die Wasserphase abgetrennt und mit basischem Ionenaustauscher (OH-Form) auf pH 10 gebracht. Nach Abfiltrieren des Ionenaustauschers und Eindampfen des Filtrats im Vakuum bleibt das Produkt als farbloser Schaum zurück.

$R_f$ = 0,52 ($CH_2Cl_2/CH_3OH$/20% wässriges $NH_3$ = 2/4/1).

*Beispiel 10*

*1-N-(4-Hydroxycyclohex-1-yl)-sisomicin*

110 mg 2',3,3'',6'-Tetra-N-(o-nitrophenylsulfenyl)-sisomicin in 1,5 ml Tetrahydrofuran/0,45 ml Wasser werden mit 150 mg 4-Hydroxycyclohexanon und 125 mg $NaBH_3CN$ versetzt und mit Essigsäure auf pH 5 gebracht. Nach 1 Stunde wird der Ansatz wie unter Beispiel 9 beschrieben auf das Endprodukt hin aufgearbeitet.

$R_f$ = 0,45 ($CH_2Cl_2/CH_3OH$/20% wässriges $NH_3$ = 2/4/1).

*Beispiel 11*

*1-N-(Piperidin-4-yl)-sisomicin*

1 g 3''-N-(o-Nitrophenylsulfenyl)-2',3,6'-tris-N-trichloracetylsisomicin wird in 4,5 ml Tetrahydrofuran und 1,5 ml Wasser gelöst, mit 270 mg 4-Piperidon und 300 mg $NaBH_3CN$ versetzt und mit Essigsäure auf pH 5 gebracht. Nach 48 Stunden bei Raumtemperatur wird der Ansatz eingedampft, in 8 ml $CH_3OH$ gelöst, mit 4 ml 4n KOH 2 Stunden bei Raumtemperatur stehen gelassen und im Vakuum erneut eingedampft. Der Rückstand wird mit 640 mg 2-Mercaptobenzthiazol in 2,25 ml $CH_3OH$ und 3,8 ml $CH_2Cl_2$ versetzt, mit konz. $HCl/CH_3OH$ = 1/1 auf pH 1 gebracht, mit 10 ml $H_2O$ versetzt, die Wasserphase abgetrennt und mit 2n NaOH auf pH 8 gebracht. Nach dem Eindampfen im Vakuum wird der Ansatz an Kieselgel chromatographiert und das Produkt mit dem $R_f$-Wert = 0,44 (Laufmittel: $CH_2Cl_2/CH_3OH$/20% wässriges $NH_3$ = 2/4/1) isoliert.

$R_f$ = 0,44 ($CH_2Cl_2$($CH_3OH$/20% wässriges $NH_3$ = 2/4/1).

**Patentansprüche**

1. Verbindungen der allgemeinen Formel

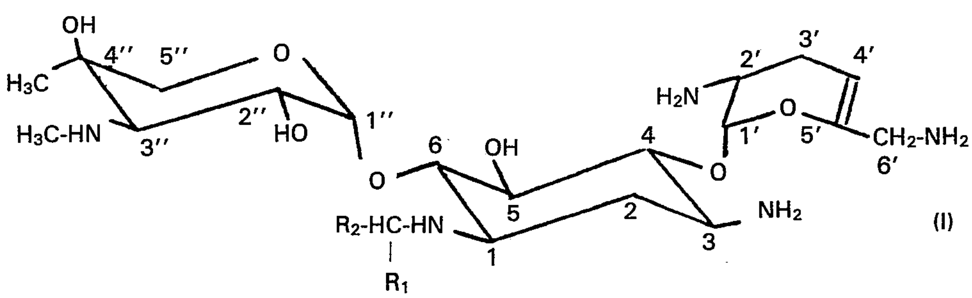

(I)

worin

$R^1$ einen durch Hydroxy, $C_1$-$C_4$-Alkoxy, Amino, $C_1$-$C_4$-Alkylamino, Di-$C_1$-$C_4$-Alkylamino, Acyloxy, Acylamino, N-$C_1$-$C_4$-Alkyl-N-acylamino, Carboxy, $C_1$-$C_4$-Alkoxycarbonyl, Aminocarbonyl, $C_1$-$C_4$-Alkylaminocarbonyl oder Di-$C_1$-$C_4$-Alkylaminocarbonyl substituierten $C_1$-$C_6$-Alkylrest und

$R^2$ einen gegebenenfalls durch Hydroxy, $C_1$-$C_4$-Alkoxy, Amino, $C_1$-$C_4$-Alkylamino, Di-$C_1$-$C_4$-Alkylamino, Acyloxy, Acylamino, N-$C_1$-$C_4$-Alkyl-N-acylamino, Carboxy, $C_1$-$C_4$-Alkoxycarbonyl, Aminocarbonyl, $C_1$-$C_4$-Alkylaminocarbonyl oder Di-$C_1$-$C_4$-Alkylaminocarbonyl substituierten $C_1$-$C_6$-Alkylrest bedeuten, oder

$R^2$-HC-$R^1$ für einen Piperidin-4-yl- oder 4-Hydroxycyclohex-1-yl-Rest steht,

mit der Bedingung, dass der Rest $R^1$-CH-$R^2$ nicht gleichzeitig Amino- und Hydroxygruppen als Substituenten tragen kann.

2. Verbindungen nach Anspruch 1, dadurch gekennzeichnet, dass $R^1$ für einen Hydroxy-substituierten $C_1$-$C_6$-Alkylrest steht.

3. Verbindungen nach Anspruch 1 oder 2, dadurch gekennzeichnet, dass $R^2$ die in Anspruch 1 für $R^1$ angegebene Bedeutung besitzt.

4. Verbindungen nach Anspruch 1 bis 3, dadurch gekennzeichnet, dass $R^2$ einen gegebenenfalls durch Hydroxy oder Carboxy substituierten $C_1$-$C_6$-Alkylrest bedeutet.

5. Verfahren zur Herstellung von Verbindungen nach Anspruch 1, dadurch gekennzeichnet, dass man Verbindungen der Formel

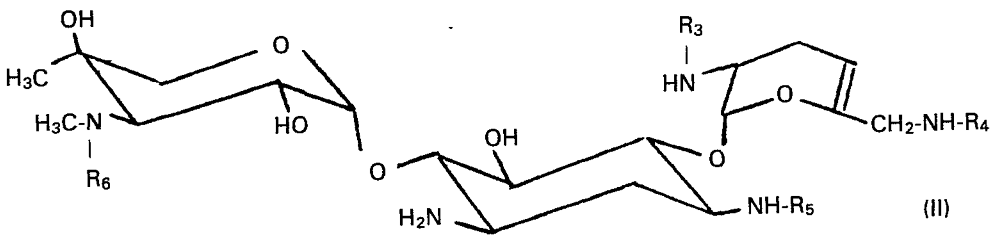

(II)

worin

$R_3$, $R_4$, $R_5$ und $R_6$ -SR' oder CO-R'' bezeichnen, wobei

R' gegebenenfalls substituiertes Phenyl, Di- oder Triphenylmethyl und

$$R'' \ -(CH_2)_{n_1}\text{-B oder -OC-}\begin{cases}(CH_2)_{n_2}\text{-B}\\(CH_2)_{n_3}\text{-H}\\(CH_2)_{n_4}\text{-H}\end{cases}$$

worin

B Wasserstoff oder gegebenenfalls substituiertes Phenyl bedeuten und

$n_1$, $n_2$, $n_3$ und $n_4$ unabhängig voneinander für eine Zahl von 0 bis 5 stehen,

mit einer Carbonylverbindung der Formel

$$R_2\text{-}\overset{\overset{\large O}{\|}}{C}\text{-}R_1 \qquad (III)$$

worin

$R_1$ und $R_2$ die in Anspruch 1 bis 4 angegebene Bedeutung haben,

und wobei Amino und Alkylamino als Substituenten von $R_1$ und $R_2$ durch R'-S- oder R''-CO-Gruppen geschützt sind, in Gegenwart eines Wasserstoff-Donor-Reduktionsmittels umsetzt und anschliessend die Schutzgruppen -S-R' oder -CO-R'' abspaltet.

6. Verbindungen gemäss Anspruch 1 bis 4 zur Verwendung bei der Bekämpfung bakterieller Infektionen.

7. Arzneimittel, gekennzeichnet durch einen Gehalt an wenigstens einer Verbindung gemäss Anspruch 1 bis 4.

8. Antibakterielles Arzneimittel, gekennzeichnet durch einen Gehalt an wenigstens einer Verbindung gemäss Anspruch 1 bis 4.

9. 1-N-(1-Hydroxyprop-2-yl)-sisomicin.

10. 1-N-(1,3-Dihydroxy-prop-2-yl)-sisomicin.

## Revendications

1. Composés de formule générale:

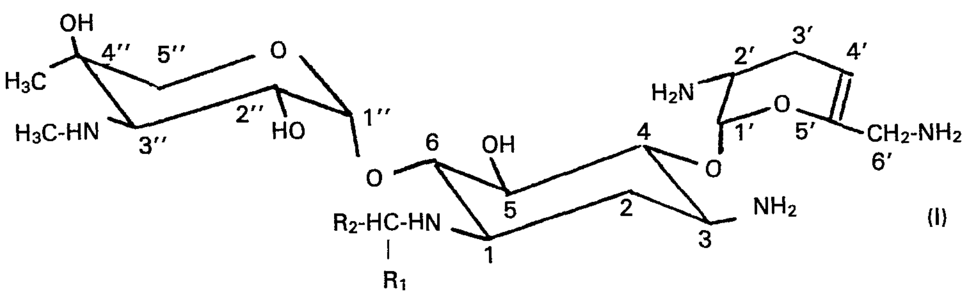

(I)

dans laquelle

$R^1$ représente un groupe alkyle en $C_1$-$C_6$ substitué par un groupe hydroxy, un groupe alcoxy en $C_1$-$C_4$, un groupe amino, un groupe alkyl(en $C_1$-$C_4$)amino, un groupe di-alkyl(en $C_1$-$C_4$)amino, un groupe acyloxy, un groupe acylamino, un groupe N-alkyl(en $C_1$-$C_4$)-N-acylamino, un groupe carboxy, un groupe alcoxy(en $C_1$-$C_4$)carbonyle, un groupe aminocarbonyle, un groupe alkyl(en $C_1$-$C_4$)aminocarbonyle ou par un groupe di-alkyl(en $C_1$-$C_4$)aminocarbonyle, et

$R^2$ représente un groupe alkyle en $C_1$-$C_6$ éventuellement substitué par un groupe hydroxy, un groupe alcoxy en $C_1$-$C_4$, un groupe amino, un groupe alkyl(en $C_1$-$C_4$)amino, un groupe di-alkyl(en $C_1$-$C_4$)amino, un groupe acyloxy, un groupe acylamino, un groupe N-alkyl(en $C_1$-$C_4$)-N-acylamino, un groupe carboxy, un groupe alcoxy(en $C_1$-$C_4$)carbonyle, un groupe aminocarbonyle, un groupe alkyl(en $C_1$-$C_4$)aminocarbonyle ou par un groupe di-alkyl(en $C_1$-$C_4$)aminocarbonyle, ou

$R^2$-HC-$R^1$ (avec liaison libre au C) représente un groupe pipéridin-4-yle ou un groupe 4-hydroxy-cyclohex-1-yle,

avec cette condition que le radical $R^1$-CH-$R^2$ ne peut comporter simultanément des groupes amino et hydroxy comme substituants.

2. Composés suivant la revendication 1, caractérisés en ce que $R^1$ représente un groupe alkyle en $C_1$-$C_6$ substitué par un groupe hydroxy.

3. Composés suivant la revendication 1 ou 2, caractérisés en ce que $R^2$ a la signification indiquée pour $R^1$ dans a revendication 1.

4. Composés suivant les revendications 1 à 3, caractérisés en ce que $R^2$ représente un groupe alkyle en $C_1$-$C_6$ éventuellement substitué par un groupe hydroxy ou par un groupe carboxy.

5. Procédé de préparation de composés suivant la revendication 1, caractérisé en ce qu'on fait réagir les composés de formule:

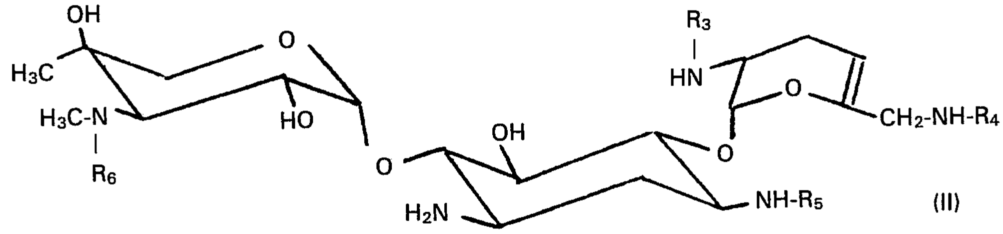

(II)

dans laquelle

$R_3$, $R_4$, $R_5$ et $R_6$ représentent chacun -SR' ou CO-R'',

R' représentant un groupe triphénylméthyle, un groupe diphénylméthyle ou un groupe phényle éventuellement substitué, et

R'' représentant

R'' -$(CH_2)_{n_1}$-B ou 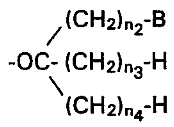

où

B représente un atome d'hydrogène ou un groupe phényle éventuellement substitué, et

$n_1$, $n_2$, $n_3$ et $n_4$ représentent chacun indépendamment l'un de l'autre un nombre de 0 à 5, avec un composé carbonyle de formule:

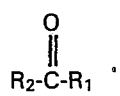

(III)

dans laquelle

$R_1$ et $R_2$ ont les significations indiquées dans les revendications 1 à 4,

les groupes amino et alkylamino, en tant que substituants de $R_1$ et de $R_2$, étant protégés par des groupes R'-S- ou R''-CO-, en présence d'un agent réducteur donneur d'hydrogène, puis on sépare les groupes protecteurs -S-R' ou -CO-R''.

6. Composés suivant les revendications 1 à 4 en vue de les utiliser pour combattre les infections bactériennes.

7. Médicament, caractérisé en ce qu'il contient au moins un composé suivant les revendications 1 à 4.

8. Médicament antibactérien, caractérisé en ce qu'il contient au moins un composé suivant les revendications 1 à 4.

9. La 1-N-(1-hydroxyprop-2-yl)-sisomycine.

10. La 1-N-(1,3-dihydroxy-prop-2-yl)-sisomycine.

**Claims**

1. Compounds of the general formula

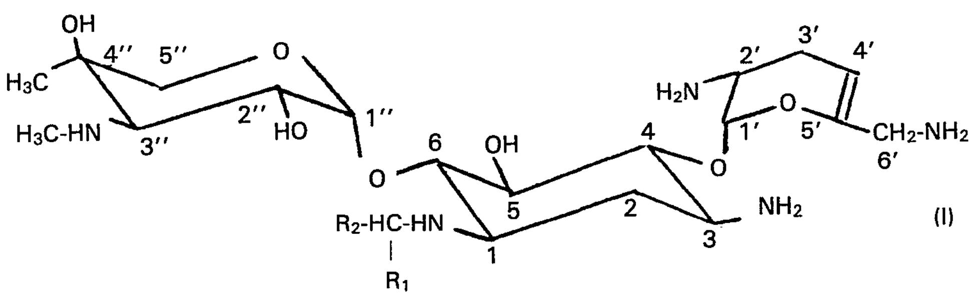

wherein

$R^1$ denotes a $C_1$-$C_6$-alkyl radical which is substituted by hydroxy, $C_1$-$C_4$-alkoxy, amino, $C_1$-$C_4$-alkylamino, di-$C_1$-$C_4$-alkylamino, acyloxy, acylamino, N-$C_1$-$C_4$-alkyl-N-acylamino, carboxyl, $C_1$-$C_4$-alkoxycarbonyl, aminocarbonyl, $C_1$-$C_4$-alkylaminocarbonyl or di-$C_1$-$C_4$-alkylaminocarbonyl and

$R^2$ denotes a $C_1$-$C_6$-alkyl radical which is optionally substituted by hydroxy, $C_1$-$C_4$-alkoxy, amino, $C_1$-$C_4$-alkylamino, di-$C_1$-$C_4$-alkylamino, acyloxy, acylamino, N-$C_1$-$C_4$-alkyl-N-acylamino, carboxyl, $C_1$-$C_4$-alkoxycarbonyl, aminocarbonyl, $C_1$-$C_4$-alkylaminocarbonyl or di-$C_1$-$C_4$-alkylaminocarbonyl, or

$R^2$-HC-$R^1$ represents a piperidin-4-yl or 4-hydroxycyclohex-1-yl radical,

with the condition that the radical $R^1$-CH-$R^2$ cannot simultaneously carry amino groups and hydroxyl groups as substituents.

2. Compounds according to claim 1, characterised in that $R^1$ represents a hydroxyl-substituted $C_1$-$C_6$-alkyl radical.

3. Compounds according to claim 1 or 2, characterised in that $R^2$ has the meaning given for $R^1$ in claim 1.

4. Compounds according to claim 1 to 3, characterised in that $R^2$ denotes a $C_1$-$C_6$-alkyl radical which is optionally substituted by hydroxyl or carbonyl.

5. Process for the preparation of compounds according to claim 1, characterised in that compounds of the formula

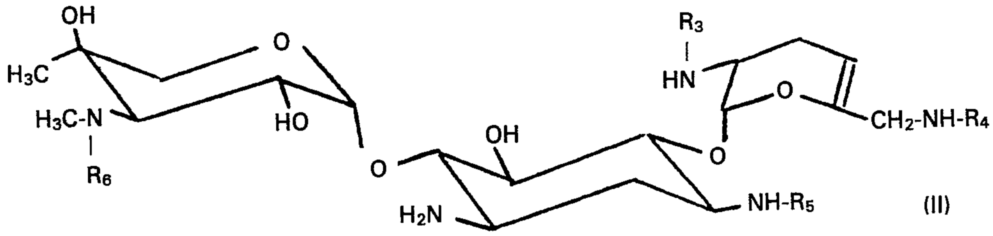

(II)

wherein

$R_3$, $R_4$, $R_5$ and $R_6$ designate -SR' or CO-R'',

wherein

R' optionally substituted phenyl or di- or triphenylmethyl and

$R''$ $-(CH_2)_{n_1}-B$ or 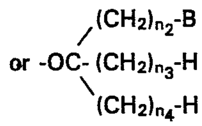

wherein

B denotes hydrogen or optionally substituted phenyl and

$n_1$, $n_2$, $n_3$ and $n_4$ independently of one another represent a number from 0 to 5,

are reacted with a carbonyl compound of the formula

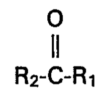

(III)

wherein

$R_1$ and $R_2$ have the meaning indicated in claim 1 to 4, and wherein amino and alkylamino as substituents of $R_1$ and $R_2$ are protected by R'-S- or R''-CO-groups, in the presence of a hydrogen donor reducing agent and the protective groups -S-R' or -CO-R'' are then split off.

6. Compounds according to claim 1 to 4 for use in combating bacterial infections.

7. Medicaments, characterised in that they contain at least one compound according to claim 1 to 4.

8. Antibacterial medicament, characterised in that it contains at least one compound according to claim 1 to 4.

9. 1-N-(1-Hydroxyprop-2-yl)-sisomicin.

10. 1-N-(1,3-Dihydroxy-prop-2-yl)-sisomicin.